# EUROPEAN PATENT SPECIFICATION

(11) **EP 3 658 175 B1**
(45) Date of publication and mention of the grant of the patent: **05.04.2023**
(21) Application number: 18753249.4
(22) Date of filing: 24.07.2018
(51) Int. Cl.: A61K 38/40, A61K 31/122, A61K 31/593, A61K 31/726, A23L 33/125, A23L 33/15, A23L 33/155, A23L 33/19, A61P 19/10

(54) **COMPOSITION FOR THE PREVENTION AND TREATMENT OF OSTEOPENIA**
ZUSAMMENSETZUNG ZUR PRÄVENTION UND BEHANDLUNG VON OSTEOPENIE
COMPOSITION DESTINÉE À LA PRÉVENTION ET AU TRAITEMENT DE L'OSTÉOPÉNIE

(30) Priority: 25.07.2017 IT 201700084683
(43) Date of publication of application: 03.06.2020
(73) Proprietor: Sochim International S.p.A., 20010 Cornaredo (MI) (IT)
(72) Inventor: EIGENMANN, Carolina, 20010 Cornaredo (MI) (IT)
(74) Representative: Villa, Livia
(86) International application number: PCT/IB2018/055483
(87) International publication number: WO 2019/021163

(56) References cited:
- WO-A1-2008/009798
- WO-A1-2011/074992
- CN-A- 101 156 630
- JP-A- 2005 068 060
- JP-A- 2006 083 151
- US-A1- 2007 253 941
- US-A1- 2015 174 218
- LANHAM-NEW SUSAN A: "Importance of calcium, vitamin D and vitamin K for osteoporosis prevention and treatment", PROCEEDINGS OF THE NUTRITION SOCIETY, LONDON, GB, vol. 67, 1 May 2008 (2008-05-01), pages 163-176, XP002573405, ISSN: 0029-6651, DOI: 10.1017/S0029665108007003 [retrieved on 2008-04-15]
- CORNISH J ET AL: "Lactoferrin is a potent regulator of bone cell activity and increases bone formation in vivo", ENDOCRINOLOGY, THE ENDOCRINE SOCIETY, US, vol. 145, no. 9, 1 January 2004 (2004-01-01), pages 4366-4374, XP003011980, ISSN: 0013-7227, DOI: 10.1210/EN.2003-1307
- H. Y. GUO ET AL: "Orally Administered Lactoferrin Preserves Bone Mass and Microarchitecture in Ovariectomized Rats", THE JOURNAL OF NUTRITION, vol. 139, no. 5, 25 March 2009 (2009-03-25), pages 958-964, XP055183550, ISSN: 0022-3166, DOI: 10.3945/jn.108.100586

## Description

### FIELD OF THE INVENTION

The present invention relates to a composition for the prevention and treatment of osteopenia, as well as for the prevention of osteoporosis. In particular, the composition of the invention comprises a synergistic association of components, namely lactoferrin, chondroitin sulfate, vitamin D and vitamin K, which has been shown to be significantly active in promoting bone metabolism.

### BACKGROUND ART

Osteopenia is defined as the reduction of bone mass due to inadequate bone matrix synthesis, i.e. the bone condition wherein the mineral density is below normal values, but cannot yet be considered manifest osteoporosis, as there is no evidence of fracture.

The progressive bone loss linked to aging, due to the prevalence of the bone resorption process over the formation of new bone, leads to a subclinical condition known as osteopenia, i.e. a situation of bone fragility which can be interpreted as the prelude of the pathological condition of osteoporosis. Osteoporosis is characterised by a reduction in and qualitative alterations of bone mass (progressive deterioration of the macro and microarchitecture of the bone tissue), which leads to an increased fragility and consequent increase in the risk of fracture. On average, the beginning of the bone loss process occurs after what is known as the "bone mass peak" is reached, i.e. the stage of maximum increase in bone mineral density, at the age of around 30-35 years. After said age, the decremental stage begins and continues throughout the rest of life.

Osteopenia can also result from one or more other conditions, diseases, or treatments. Women are more likely to develop osteopenia and osteoporosis than men. Indeed, in women, the decrease is more precocious, as the onset thereof coincides with the menopause and continues exponentially thereafter.

In both men and women, however, the following factors are known to accelerate the progression of osteopenia:
- eating disorders or metabolic problems that do not allow the body to assimilate and use sufficient vitamins and minerals,
- chemotherapy treatments, or treatments with drugs, such as steroids, which are used to treat a number of conditions, including asthma,
- exposure to radiation.

Having a family history of osteoporosis, being overly thin, being Caucasian or Asian, getting little exercise, smoking, and over-consuming alcoholic beverages are further factors that increase the risk of osteopenia and, possibly, osteoporosis.

Osteopenia is asymptomatic, there is no pain or change, since the bone simply becomes more fragile and therefore more susceptible to fractures.

Osteopenia is typically treated by introducing lifestyle changes in order to prevent or in any case slow down bone mass loss and deter the onset of osteoporosis. At the same time, intake of supplements containing calcium and/or vitamin D is recommended.

US 2007253941 and US2015174218 disclose compositions which include coenzyme Q10 and angiogenin, optionally with chondroitin sulfate, lactoferrin and/or vitamins, for bone health formulations.

WO2008009798 discloses compositions for preventing and/or treating degenerative joint diseases, comprising, as active components, an effective amount of a mixture of D-glucosamine, of lactoferrin and of chondroitin sulfate.

WO2011074992 discloses the combination of glucosamine, lactoferrin and chondroitin sulfate with anti-inflammatory activity.

CN101156630 discloses an arthrosis strengthening milk containing glucosamine sulphate, soybean isoflavones, creatine, chondroitin sulfate and lactoferrin. Lanham-New, S. (Proceedings of the Nutrition Society, 67(2), 163-176; 2008) discusses the importance of micronutrients, such as calcium, vitamin D and vitamin K, for osteoporosis prevention and treatment.

In severe cases, drugs to counteract bone density loss are prescribed, such as bisphosphonates, raloxifene, and hormone replacement therapy.

Given the widespread nature of said bone condition, as well as the occurrence thereof among elderly subjects, the problem faced is the provision of an effective remedy, which is also practical to use, to slow down the process of bone density reduction, and thereby desirably postponing the need of drugs for as long as possible.

An object of the present invention is therefore to provide such a remedy.

### SUMMARY OF THE INVENTION

Said object has been achieved by a composition as stated in Claim 1.

In a further aspect, the present invention relates to the use of said composition for the prevention and treatment of osteopenia, as well as the use of said composition as an adjuvant in the prevention of osteoporosis.

In a further aspect, the present invention relates to a food supplement comprising said composition. The scope of the invention is defined by the claims. The references to methods of treatment in the summary and detailed description of the invention in this description are to be interpreted as references to the compounds, pharmaceutical compositions and medicaments of the present invention for use in a method for treatment of the human (or animal) body by therapy.

### BRIEF DESCRIPTION OF THE FIGURES

The characteristics and advantages of the present invention will become apparent from the following detailed description, the embodiments provided by way of non-limiting examples and the figures annexed hereto, wherein:
- Figures 1-3 show the measurement, at 3, 5, and 7 days respectively, of fold expression by SaOS2 osteoblastic cells in the presence of two different concentrations of Vitamin K2 (menaquinone), i.e. 5 µM and 10 µM, on machined and DAE surfaces, as discussed in Example 12;
- Figures 4-6 show the comparison between the measurement, at 3, 5, and 7 days respectively, of fold expression by SaOS2 cells in the presence of Vitamin K2 (menaquinone) and of the Product according to the invention, using the same concentrations as in Figures 1-3, as discussed in Example 12;
- Figures 7 and 8 show the 7-day comparison between the control and the cells cultured in the presence of the Product, observed by fluorescence microscopy, as discussed in Example 12; and
- Figure 9 shows the measurement of specific alkaline phosphatase (or "Alp"), i.e. the amount of said enzyme produced by each cell, obtained after 3 days from cells cultured on the machined surface, as discussed in Example 12.

### DETAILED DESCRIPTION OF THE INVENTION

Therefore, the invention relates to a composition comprising lactoferrin, chondroitin sulfate, vitamin D and vitamin K.

Lactoferrin, also known as lactotransferrin, is a multifunctional globular protein having antimicrobial activity which is both bactericidal and fungicidal. Lactoferrin belongs to the transferrin family and has a molecular mass of 80 KDa, with two ferric ion binding sites (Fe³⁺), similar to said transferrin.

Chondroitin sulfate is a glycosaminoglycan (GAG) sulfate, composed of an alternating sugar chain (N-acetylgalactosamine and glucuronic acid). It is normally found associated with proteins, to form a proteoglycan. A chondroitin chain can have over 100 sugars, each of which can bind sulfate ions, in variable positions and amounts.

It has surprisingly been found that, in the compositions in which lactoferrin is present in association with chondroitin sulfate, a significant synergistic effect is obtained which determines the rebalancing of bone metabolism, thereby promoting anabolic activity in bones, as will be discussed in more detail below.

Preferably, in the composition of the invention, lactoferrin is in a higher amount than chondroitin sulfate.

More preferably, lactoferrin and chondroitin sulfate are in weight ratio of at least 1.2:1. Even more preferably, lactoferrin and chondroitin sulfate are in weight ratio not higher than 100:1.

In preferred embodiments, lactoferrin and chondroitin sulphate are in a weight ratio of 1.5:1 to 50:1.

In particularly preferred embodiments, lactoferrin and chondroitin sulphate are in a weight ratio of 1.5: 1 to 10:1, more preferably 1.5: 1 to 5:1.

In certain embodiments, the composition of the invention comprises up to 30 wt% of lactoferrin, and more preferably up to 20 wt%.

For the purposes of the present invention, unless otherwise specified, "wt%" means % by weight based on the weight of the composition of the invention.

In other embodiments, the composition of the invention comprises up to 20 wt% of chondroitin sulfate, and more preferably up to 15 wt%.

The composition of the invention can further comprise an adjuvant selected from collagen, hydrolysed collagen type I, hydrolysed collagen type II, hyaluronic acid, dermatan sulfate, keratan sulfate, heparin, heparan sulfate, and mixtures thereof.

Said adjuvant advantageously supports the action of chondroitin sulfate in bone remodelling.

Preferably, the composition of the invention comprises an adjuvant selected from collagen, hydrolysed collagen type II, hyaluronic acid, and mixtures thereof.

In preferred embodiments, the composition of the invention comprises up to 60 wt% of adjuvant, more preferably up to 45 wt%.

In preferred embodiments, lactoferrin, chondroitin sulphate, and optionally said adjuvant, are the only active components present in the composition of the invention, wherein "active" means that said components play an active role in the prevention and treatment of osteopenia, the other components being pharmaceutically acceptable excipients.

The composition of the invention comprises up to 0.05 wt% of vitamin D, more preferably up to 0.01 wt%.

"Vitamin D" means a group of liposoluble pro-hormones consisting of vitamin D1, vitamin D2, vitamin D3, vitamin D4, vitamin D5, or a mixture thereof.

Preferably, the vitamin D is vitamin D3. Indeed, studies have found Vitamin D3, or cholecalciferol, to be the most effective form of vitamin D to ensure proper concentrations of said nutrient in the blood.

The composition of the invention comprises up to 0.05 wt% of vitamin K, more preferably up to 0.01 wt%.

"Vitamin K" means a group of vitamins consisting of vitamin K1, vitamin K2, and vitamin K3.

Preferably, the vitamin K is vitamin K2. Indeed, said vitamin plays a fundamental role in calcium homeostasis. Vitamin K is the cofactor of the enzyme responsible for the gamma-carboxylation process, which activates certain proteins known as "GLA proteins".

One of these proteins is osteocalcin, a protein involved in the bone mineralisation process. Therefore, vitamin K2 is essential to prevent calcium from depositing on the vascular endothelium and is, instead, efficiently transported and deposited on the bone.

As will be seen in the examples below, the composition of the invention which further comprises vitamin K has shown a remarkable stimulation effect on the expression of genes of relevance for osteogenesis and the subexpression of pro-osteoclastogenic RANKL, i.e. an imbalance in the OPG/RANKL ratio. Moreover, it was observed that, at the concentrations used, the association of lactoferrin and vitamin K in particular reduces the number of cells adhering to the titanium substrate employed and alters the morphology thereof, steering said cells towards greater differentiation. It has also been observed that, at the concentrations assessed, the effect of said association is not related to the surface finish of the titanium substrate, whereas in the case of vitamin K alone, greater efficacy was observed on the DAE (i.e. Double Acid Etching) surface, in addition to a concentration dependence.

The composition of the invention comprises lactoferrin, chondroitin sulfate, and vitamin K.

In particularly preferred embodiments, the composition of the invention comprises said adjuvant, further to vitamin D, and vitamin K.

It should be understood that the aspects identified as preferred for the individual components, i.e. said adjuvant, vitamin D and vitamin K, have likewise to be considered preferred in embodiments which include a combination thereof.

In certain embodiments, the composition of the invention consists essentially of lactoferrin, chondroitin sulfate, said adjuvant, vitamin D, and vitamin K. The expression "consists essentially of" means that lactoferrin, chondroitin sulfate, said adjuvant, vitamin D, and vitamin K are the only ingredients present in the composition of the invention which play an active role in the treatment of osteopenia, while any other components or excipients do not interfere with the action of said active ingredients and can be mixed therewith.

In other embodiments, the composition of the invention consists of lactoferrin, chondroitin sulfate, said adjuvant, vitamin D, and vitamin K and pharmaceutically acceptable excipients.

In preferred embodiments, the composition of the invention is in the form of a unit dose. Preferably, said unit dose comprises 1-1000 mg of lactoferrin, more preferably 20-500 mg.

In particularly preferred embodiments, said unit dose comprises 50-200 mg of lactoferrin. Preferably, said unit dose comprises 0.1-800 mg of chondroitin sulfate, more preferably 0.5-400 mg.

In particularly preferred embodiments, said unit dose comprises 1-100 mg of chondroitin sulfate.

Preferably, said unit dose further comprises up to 2200 UI of vitamin D, more preferably 1500-2100 UI. In particularly preferred embodiments, said unit dose further comprises 2000 UI of vitamin D.

Preferably, said unit dose further comprises 1-500 µg of vitamin K, more preferably 1-200 µg.

Preferably, said unit dose further includes up to 400 mg of said adjuvant.

In particularly preferred embodiments, said unit dose further comprises 50-300 mg of said adjuvant.

In preferred embodiments, said unit dose comprises 1-1000 mg of lactoferrin, 0.1-800 mg of chondroitin sulfate, up to 400 mg of said adjuvant, up to 2200 UI of vitamin D, and 1-500 µg of vitamin K.

In particularly preferred embodiments, said unit dose comprises 20-500 mg of lactoferrin, 0.5-400 mg of chondroitin sulfate, 50-300 mg of said adjuvant, 1500-2100 UI of vitamin D, and 1-200 µg of vitamin K.

Further preferred are the compositions, as well as the unit doses, comprising:

| | |
|---|---|
| - lactoferrin | 50-200 mg |
| - chondroitin sulfate | 1-100 mg |
| - adjuvant | 50-300 mg |
| - Vitamin D3 | 1900-2100 UI |
| - Vitamin K2 | 1-200 µg |

It should be understood that the preferred aspects specified for the individual components should likewise be considered preferred in the unit dose embodiments stated above.

The composition of the invention, also in the form of a unit dose, may further comprise calcium, magnesium, potassium, arginine, histidine, isoleucine, leucine, lysine, methionine, phenylalanine, threonine, tryptophan, valine, alanine, aspartic acid, cysteine, glutamic acid, glycine, hydroxyproline, proline, serine, tyrosine, and mixtures thereof. The composition of the invention, also in the form of a unit dose, may further comprise pharmaceutically acceptable excipients. The term "excipient" means a compound or a mixture thereof suitable for use in a formulation for the prevention and treatment of osteopenia. For example, an excipient for use in a pharmaceutical formulation should not generally cause an adverse response in a patient, nor should it significantly inhibit the efficacy of the composition.

Suitable excipients include acidifiers, acidity regulators, anti-caking agents, antioxidants, bulking agents, resistance agents, gelling agents, coating agents, modified starches, sequestering agents, thickeners, sweeteners, diluents, disaggregating agents, glidants, colorants, binders, lubricants, stabilisers, adsorbents, preservatives, humectants, flavourings, filmogenic agents, emulsifiers, wetting agents, release retardants, and mixtures thereof.

The addition of excipients may be carried out by using commonly methods known in the art. Indeed, the components can, for example, be mixed as such or with one or more excipients, either sealed in soft-gel capsules or in solid form, such as a tablet, mini-tablet, micro-tablet, granule, micro-granule, pellet, multiparticulate, micronised particulate, powder, or in the form of a solution, emulsion, gel, vials, or of drops or sprays.

In preferred embodiments, the composition of the invention is in the form of soft-gel capsules comprising a lipophilic matrix, said lipophilic matrix comprising medium-chain triglycerides, food-grade gelatin, soya glycerol phospholipids, propylene glycol esters, succinate gelatin, soy lecithin, phosphatidylcholine in medium-chain triglycerides, corn starch, sorbitol, mono- and diglycerides of fatty acids, polysorbitol, polyglyceryl esters of fatty acids, beeswax, silicon dioxide, sunflower lecithin, sunflower oil, or a mixture thereof. Indeed, the soft-gel capsules increase the absorption and therefore the bioavailability of the components, which are mostly lipophilic.

The composition of the invention may be administered via oral, nasal, sublingual, buccal, or transdermal route.

Preferably, the composition of the invention is administered via oral, sublingual or buccal route.

In a further aspect, the present invention relates to the use of said composition for the prevention and treatment of osteopenia, as well as the use as an adjuvant in the prevention of osteoporosis.

Lactoferrin acts as a growth factor and performs anabolic activity both in vitro and in vivo in bone. It preserves bone mass and bone microarchitecture and prevents bone loss. Lactoferrin induces the proliferation and differentiation of osteoblast-like cells and inhibits osteoclastogenesis. It has a double effect on both osteoblasts and osteoclasts, as well as on the OPG/RANKL/RANK pathway.

The interaction between RANK-RANKL and osteoprotegerin (OPG) is held to be decisive in the regulation of bone resorption. RANKL is a transmembrane or soluble protein, produced by cells in the stromal/osteoblastic line and activated T lymphocytes, which binds the RANK cell receptor, expressed by osteoclasts and the precursors thereof, and by dendritic cells. The interaction between RANKL and RANK stimulates the differentiation, activation, and survival of osteoclasts, with a consequent increase in bone resorption. To interrupt this circuit, it is necessary to inhibit the interaction of the RANKL with the RANK by means of the OPG, which is produced by said osteoblasts. OPG is a glycoprotein which inhibits bone resorption, which is important in the regulation of bone resorption. The OPG binds with the RANKL, as a bait receptor, and decreases the availability thereof for the RANK receptor. The OPG therefore counterbalances the biological effects of the RANKL.

It is believed that, although lactoferrin appears to have an inhibitory effect on RANKL, the former actually increases the OPG. The RANKL/OPG ratio may be one of the main mechanisms available to increase bone formation and reduce bone resorption and this aspect suggests that lactoferrin decreases osteoclast differentiation.

It is believed that also chondroitin sulfate has a role in osteoclastogenesis.

Indeed, OPG contains a heparin binding domain which binds with GAG sulfates, including therefore chondroitin sulphate. Compounds supplemented with chondroitin sulfate have been shown to promote bone remodelling and the formation of new bone with respect to chondroitin sulfate-free control composites, probably due to the possible interaction with various proteins and cytokines involved in the regulation of bone metabolism, including OPG and RANKL.

Without wishing to be bound by any theory, it is thought that the combination of lactoferrin and chondroitin sulfate of the present invention increases the anabolic activity of lactoferrin, thus significantly impacting on the OPG/RANKL/RANK pathway and, consequently, on osteoclastogenesis, promoting osteoblastogenesis and therefore the anabolic activity of the bone metabolism.

Preferably, said composition for use in the prevention and treatment of osteopenia is administered in the form of a unit dose as described above, 1-5 times a day, more preferably 1-3 times a day, even more preferably once a day.

Preferably, said composition is administered in an amount so as to provide 1-1000 mg of lactoferrin and 0.1-800 mg of chondroitin sulfate per day.

More preferably, said composition is administered in such amounts as to provide 20-500 mg of lactoferrin and 0.5-400 mg of chondroitin sulfate per day.

Even more preferably, said composition is administered in an amount so as to provide 50-200 mg of lactoferrin and 1-100 mg of chondroitin sulfate per day.

In a further aspect, the present invention relates to a food supplement comprising this composition.

This food supplement can be used in the prevention and treatment of osteopenia, as well as an adjuvant in the prevention of osteoporosis.

It should be understood that all the possible combinations of preferred aspects of the components of the composition, uses and food supplement as above reported, are to be deemed as hereby disclosed.

It should be understood also that all the aspects identified as preferred and advantageous for the composition and its components are to be deemed as similarly preferred and advantageous also for the uses thereof and the food supplement.

Below are working examples of the present invention provided for illustrative purposes.

### EXAMPLES

### Example 1.

A composition was prepared comprising:

| | |
|---|---|
| - lactoferrin | 100 mg |
| - chondroitin sulfate | 50 mg |
| - hyaluronic acid | 25 mg |
| - hydrolysed collagen type II | 150 mg |
| - Vitamin D3 | 2000 UI |
| - Vitamin K2 | 45 µg |

The composition was subsequently formulated in a 1200 mg soft-gel capsule comprising sunflower oil, food-grade gelatin, mono- and diglycerides of fatty acids, and sunflower lecithin.

### Example 2.

A composition was prepared comprising:

| | |
|---|---|
| - lactoferrin | 180 mg |
| - chondroitin sulfate | 60 mg |
| - hyaluronic acid | 30 mg |
| - hydrolysed collagen type II | 180 mg |
| - Vitamin D3 | 2000 UI |
| - Vitamin K2 | 50 µg |

### Example 3.

A composition was prepared comprising:

| | |
|---|---|
| - lactoferrin | 100 mg |
| - chondroitin sulfate | 50 mg |
| - Vitamin D3 | 2000 UI |
| - Vitamin K2 | 45 µg |

### Example 4 (reference example)

A composition was prepared comprising:

| | |
|---|---|
| - lactoferrin | 100 mg |
| - chondroitin sulfate | 50 mg |

The composition was subsequently formulated in a 1200 mg soft-gel capsule comprising sunflower oil, food-grade gelatin, mono- and diglycerides of fatty acids, and sunflower lecithin.

### Example 5 (reference example)

A composition was prepared comprising:

| | |
|---|---|
| - lactoferrin | 100 mg |
| - chondroitin sulfate | 100 mg |

The composition was subsequently formulated in a 1200 mg soft-gel capsule comprising sunflower oil, food-grade gelatin, mono- and diglycerides of fatty acids, and sunflower lecithin.

### Example 6 (reference example)

A composition was prepared comprising:

| | |
|---|---|
| - lactoferrin | 80 mg |
| - chondroitin sulfate | 100 mg |

The composition was subsequently formulated in a 1200 mg soft-gel capsule comprising sunflower oil, food-grade gelatin, mono- and diglycerides of fatty acids, and sunflower lecithin.

### Example 7.

A composition was prepared comprising:

| | |
|---|---|
| - lactoferrin | 100 mg |
| - chondroitin sulfate | 100 mg |
| - Vitamin D3 | 2000 UI |
| - Vitamin D2 | 45 µg |

The composition was subsequently formulated in a 1200 mg soft-gel capsule comprising sunflower oil, food-grade gelatin, mono- and diglycerides of fatty acids, and sunflower lecithin.

### Example 8.

A composition was prepared comprising:

| | |
|---|---|
| - lactoferrin | 80 mg |
| - chondroitin sulfate | 100 mg |
| - Vitamin D3 | 2000 UI |
| - Vitamin K2 | 45 µg |

The composition was subsequently formulated in a 1200 mg soft-gel capsule comprising sunflower oil, food-grade gelatin, mono- and diglycerides of fatty acids, and sunflower lecithin.

### Example 9.

A composition was prepared comprising:

| | |
|---|---|
| - lactoferrin | 100 mg |
| - chondroitin sulfate | 100 mg |
| - hyaluronic acid | 30 mg |
| - hydrolysed collagen type II | 180 mg |
| - Vitamin D3 | 2000 UI |
| - Vitamin D2 | 45 µg |

The composition was subsequently formulated in a 1200 mg soft-gel capsule comprising sunflower oil, food-grade gelatin, mono- and diglycerides of fatty acids, and sunflower lecithin.

### Example 10.

A composition was prepared comprising:

| | |
|---|---|
| - lactoferrin | 80 mg |
| - chondroitin sulfate | 100 mg |
| - hyaluronic acid | 50 mg |
| - hydrolysed collagen type II | 150 mg |
| - Vitamin D3 | 2000 UI |
| - Vitamin D2 | 50 µg |

The composition was subsequently formulated in a 1200 mg soft-gel capsule comprising sunflower oil, food-grade gelatin, mono- and diglycerides of fatty acids, and sunflower lecithin.

### Example 11.

A composition was prepared comprising:

| | |
|---|---|
| - lactoferrin | 250 mg |
| - chondroitin sulfate | 30 mg |
| - hyaluronic acid | 80 mg |
| - hydrolysed collagen type II | 50 mg |
| - Vitamin D3 | 2000 UI |
| - Vitamin D2 | 75 µg |

The composition was subsequently formulated in a 1200 mg soft-gel capsule comprising sunflower oil, food-grade gelatin, mono- and diglycerides of fatty acids, and sunflower lecithin.

### Example 12.

### Assessment, by RT-PCR analysis and fluorescence microscopy, of osteoblastic cell response

The purpose of this study was to measure the expression, by osteoblast-like SaOS2 cells cultured on titanium surfaces, of certain key genes involved in the osteogenesis process, by using different concentrations of the product. Furthermore, we wanted to assess, via fluorescence microscopy, the possible effects on the morphology of adhered cells.

### Materials and methods

The following materials were used:
- MenaQ7 powder P-2000, containing 2 parts per 1000 (w/w) of vitamin K2 as MK7, or vitamin K2 as menaquinone. For the sake of brevity, this material will be hereinafter referred to as "MenaQ7"
- mixture of lactoferrin and MenaQ7, produced by combining 20% MenaQ7 and 80% lactoferrin preparation, as obtained from cow's milk by freeze-drying, and containing in its turn about 90% of lactoferrin. For the sake of brevity, this formulation will be hereinafter referred to as the "Product".

The tests were performed by culturing SaOS2 osteoblast-like cells on titanium discs with different product concentrations and for different experimental times (as specified below). In particular, the titanium discs had two different surface finishes: one series of discs can be defined as "machined", i.e. it featured a turned finish (generally known as "smooth"); a second series of discs was treated to obtain a rough surface, using the Double Acid Etching (DAE) protocol. The DAE surface is used widely and represents a good part of the currently used implant surfaces.

As regards cell culture, a suspension of 7.35 ± 0.12 × 10⁴ SaOS2 cells (obtained by adding 2 mL of trypsin/EDTA solution to the monolayer contained in a T75 Falcon flask) in 2.5 mL of McCoy's 5a medium, supplemented with 15% bovine foetus serum, L-glutamine, penicillin, streptomycin and amphotericin B (all purchased from LONZA MILANO Srl, Milan) was introduced in sterile polystyrene containers with 12 compartments (12-well multiwell plates, FALCON). MenaQ7 or the Product (following solubilisation in PBS) were added to the culture medium, to achieve the desired final concentration described in the results section. The control was carried out by adding the same volume of PBS to the culture medium.

At the same time, following extraction from the sterile packs under the laminar flow hood, all nine discs were introduced for each surface type needed for this test. The containers were then placed in an incubator at 37 °C, with 5% CO₂ and 98% relative humidity, with three discs per type sampled after 72 hours, followed by a further three after 5 days, and a further three after, finally, 7 days.

At the end of each growth period, two disks for each experimental time were used for fold expression analysis, performed by real time reverse transcription PCR (qRT-PCR). The total RNA of the cells cultured on the discs was extracted after 24h, 72h, and 7 days using the MagMax Total RNA Isolation Kit (Applied Biosystems). RNA quality was assessed by checking that the A260/A280 absorbance ratio was between 1.6 and 2.0. The RNA extracted was subsequently reverse-transcribed to cDNA using the High Capacity cDNA RT kit from Applied Biosystems.

The relative quantification of genes (described in the results section) was obtained using Taq Man probes specific for each gene taken into consideration and GAPDH housekeeping fold expression levels were compared. Amplification reactions were conducted in the Step-One thermocycler (Applied Biosystems) in duplicate, following the manufacturer's instructions.

To obtain the fold expression graphs, the data was normalised using the Step-One software according to the standard ΔCt method.

One method disc per type and experimental time was subjected to fixation with 4% glutaraldehyde solution (Aldrich, Sigma, Milan) in Dulbecco's Phosphate-Buffered Saline (DPBS, Invitrogen, San Giuliano Milanese, Milan), to prepare the cells for observation by fluorescence microscopy. Following fixation, the cells were treated according to the specific protocol, the key points of which are stated below:
- permeabilisation of the cell membrane by 1% Triton X-100 solution in PBS, followed by washing in DPBS
- blocking of non-specific sites by immersion in 1% solution of Bovine Serum Albumin (BSA) and subsequent washes in DPBS;
- staining of the cell body with fluorescein-conjugated phalloidin (Alexafluor 488, Invitrogen, Milan);
- nuclei staining using DAPI dye (4', 6-Diamidino-2-Phenylindole, Dilactate) and final washings.

The samples thus prepared were observed under a Leica DMI4000B fluorescence microscope.

### Results

### Effect of MenaQ7 on fold expression by SaOS2 cells

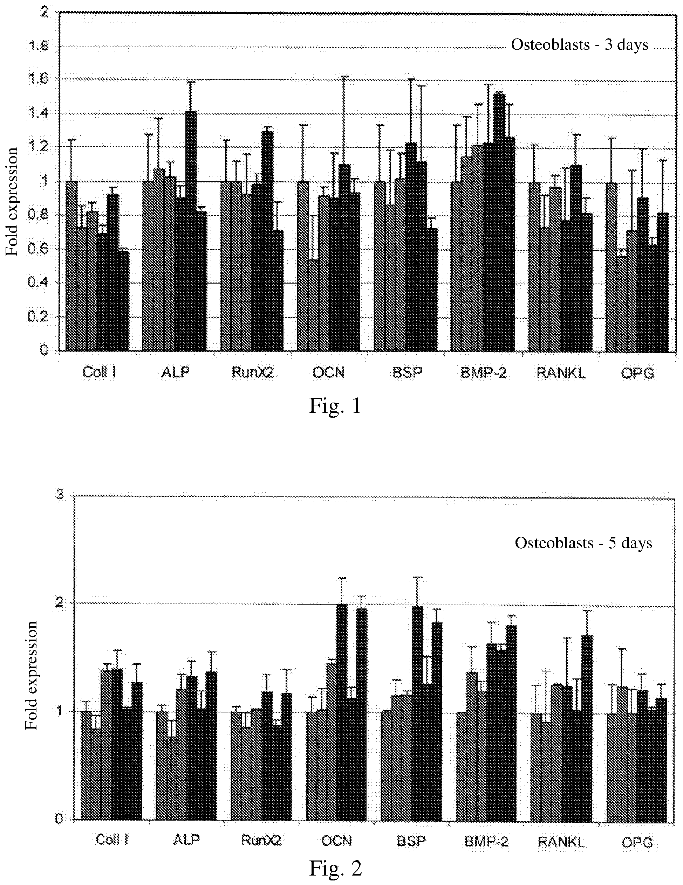

The experiment was conducted by measuring, after 3, 5 and 7 days, fold expression by SaOS2 cells in the presence of two different concentrations of MenaQ7, namely 5µM and 10 µM, on machined and DAE surfaces. The results obtained are shown in Figures 1-3, in which the light grey bars on the left-hand side refer to the machined surface, while the grey bars on the right-hand refer to the DAE surface. The order of the bars from left to right follows the concentration order, i.e. control, 5µM, and 10 µM.

The data clearly shows an overexpression of BSP, BMP-2, RUNX2, ALP and OPG by the SaOS2 cells cultured on the DAE surface.

The data also shows a concentration dependence. The effect of MenaQ7 is particularly evident on the DAE surface, suggesting cooperation between the biochemical effect of MenaQ7 and cell morphology. The DAE surface, as seen by comparing the data obtained from the two controls, already has an effect on osteogenic stimulation with respect to the machined surface. This effect is due to the fact that the roughness of the surface stimulates cell behaviour, thereby influencing the morphology, as can also be seen in the microscopy images presented below. Clearly, the morphology of the cells cultured on the DAE surface is more amenable to reception of MK7 stimuli, at least at the concentrations used. Essentially, the results of these experiments confirm the stimulating effect of MK7 on osteogenesis and suggest the interaction thereof with surface topography.

### Effect of the Product on fold expression by SaOS2 cells

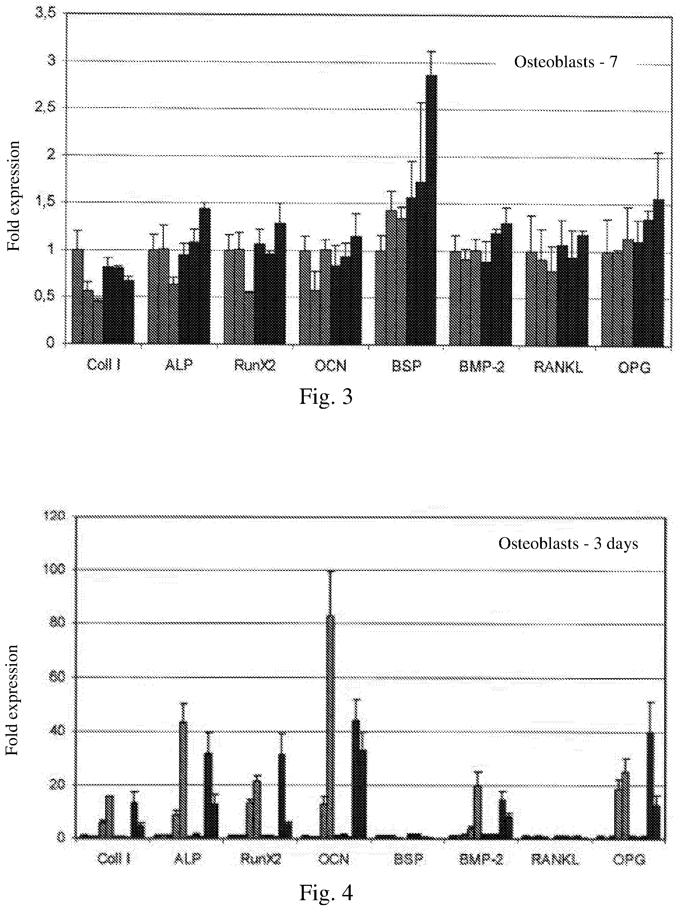

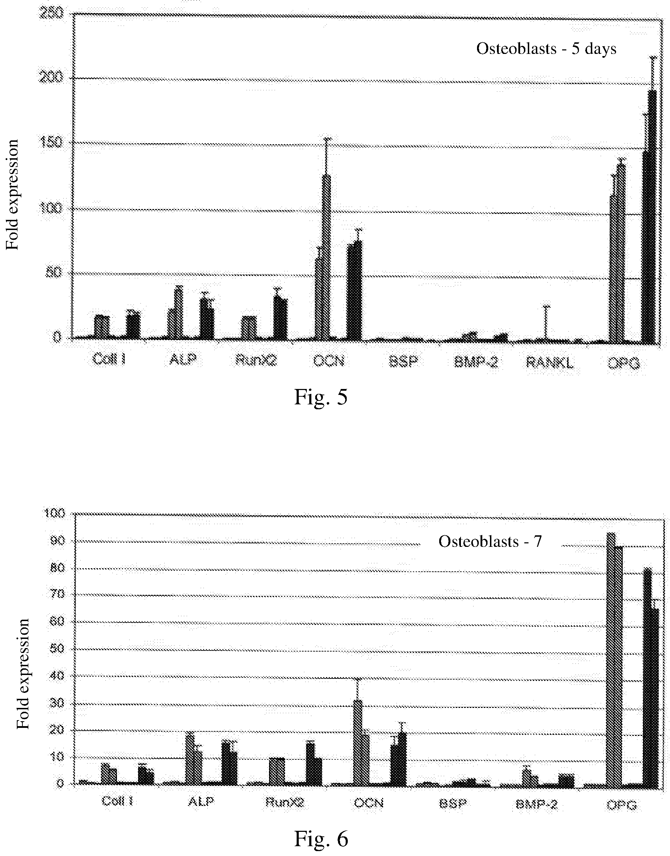

Experiments were performed by comparing the effect of the Product on SaOS2 fold expressions on the two different types of titanium surfaces. In order to highlight any synergistic effects with lactoferrin, the criterion adopted was to keep the MK7 concentration constant and then vary the total weight of the compound used, whether MenaQ7 or the Product. Using the same concentrations described above (5 µM and 10 µM), the results reported in Figures 4-6 were obtained.

The graphs show that the Product produced a very clear effect after just three days. Particularly noteworthy is the effect on osteoprotegerin, which is not accompanied by the same increase in RANKL. This data shows a clear shift in the bone formation/resorption balance towards formation, which is the typical effect on which drugs used to control osteoporosis are based. It is also interesting to note that while MenaQ7 stimulates BSP expression, the Product produces a different effect and BSP is practically the only pro-osteogenic gene not overexpressed in these experiments. This data clearly shows that lactoferrin, together with MK7, impacts significantly on the SaOS2 cells in culture.

### Effect of MenaQ7 and the Product on the morphology and number of SaOS2 cells

A series of images (not annexed) have been gathered which show the appearance of the cell layer on the machined surface and the DAE surface after 3, 5, and 7 days, on the control, with the two different concentrations of MK7 used and with the lower concentration of the Product (data for the higher concentration overlapped). In this sense, the following was observed:
- first, upon comparison to the controls, the expected effect of the superficial topography on the cellular morphology was observed. This data was clear after just 3 days and was amplified as time passed. On the machined surface, the cells always had a "fibroblastoid" appearance, i.e. elongated and aligned.
- as regards the MK7, there was a certain effect on cellular morphology, however this remained within the surface in question.

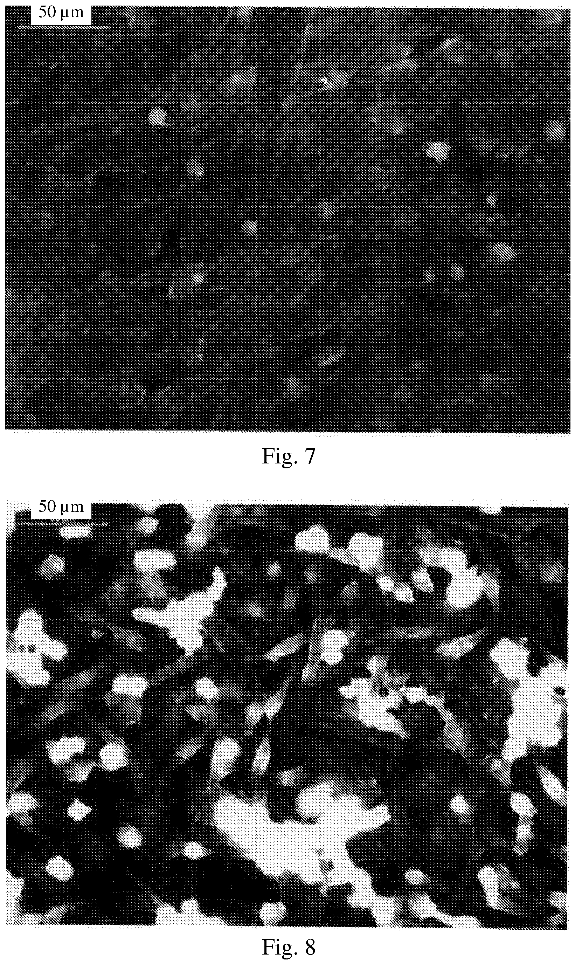

The Product showed decidedly remarkable effects, especially in terms of cell morphology. Indeed, both the cell body and the cellular organisation differed in appearance with respect to the corresponding control, tending towards a less elongated, flattened form. This was particularly evident on the machined surface, which, after 7 days, led to the formation of an elongated and aligned cell monolayer with few singularities. Generally speaking, the cellular morphology observed in the presence of the Product suggested a greater differentiation and better organisation, tending towards osteogenesis. This descriptive data was fully corroborated by the RT-PCR results described earlier. Figures 7 and 8 show the 7-day comparison between the control and the cells cultured in the presence of the Product. The presence of out-of-range and overexposed areas in Figure 8 is a direct indicator of the greater three-dimensionality of the cellular organisation observed.

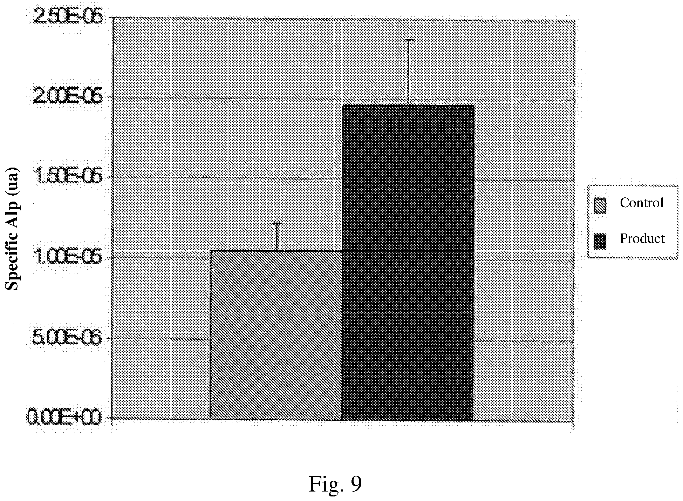

As regards the measurement of specific alkaline phosphatase (or "Alp"), i.e. the amount of this enzyme produced by each cell, Figure 9 (data obtained, after 3 days, from cells cultured on the machined surface) shows the greater activity of the cells cultured in the presence of the Product.

### Conclusions

The composition of the invention which further comprises vitamin K has shown a remarkable stimulation effect on the expression of genes of relevance for osteogenesis and the subexpression of pro-osteoclastogenic RANKL, i.e. an imbalance in the OPG/RANKL ratio. Moreover, it was observed that, at the concentrations used, the association of lactoferrin and vitamin K in particular reduces the number of cells adhering to the titanium substrate employed and alters the morphology thereof, steering said cells towards greater differentiation. It has also been observed that, at the concentrations used, the effect of said association is not related to the surface finish of the titanium substrate, whereas in the case of vitamin K alone, greater efficacy was observed on the DAE (i.e. Double Acid Etching) surface, in addition to a concentration dependence.

## Claims

1. Composition comprising lactoferrin and chondroitin sulfate, and further comprising up to 0.05 wt% of Vitamin D, and up to 0.05 wt% of Vitamin K.

2. The composition of claim 1, wherein lactoferrin and chondroitin sulfate are in a weight ratio of 1.5:1 to 50:1.

3. The composition of claim 2, wherein lactoferrin and chondroitin sulfate are in a weight ratio of 1.5:1 to 10:1, preferably 1.5:1 to 5:1.

4. The composition of any one of claims 1-3, further comprising an adjuvant selected from collagen, hydrolysed collagen type I, hydrolysed collagen type II, hyaluronic acid, dermatan sulfate, keratan sulfate, heparin, heparan sulfate, and mixtures thereof.

5. The composition of any one of claims 1-4, wherein Vitamin D is up to 0.01 wt%, on the composition weight.

6. The composition of any one of claims 1-5, wherein Vitamin K is up to 0.01 wt%, on the composition weight.

7. The composition of claim 6, in the form of a unitary dose comprising 1-1000 mg of lactoferrin, 0.1-800 mg of chondroitin sulfate, up to 400 mg of said adjuvant, up to 2200 IU of Vitamin D, and 1-500 µg of Vitamin K.

8. The composition of claim 7, in the form of a unitary dose comprising:
| | |
|---|---|
| - lactoferrin | 50-200 mg |
| - chondroitin sulfate | 1-100 mg |
| - adjuvant | 50-300 mg |
| - Vitamin D3 | 1900-2100 UI |
| - Vitamin K2 | 1-200 µg |

9. The composition of any one of claims 1-8 for use in the prevention or treatment of osteopenia, as well as for use as a coadjuvant in the prevention of osteoporosis.

10. Food supplement comprising the composition of any one of claims 1-8.

## Patentansprüche

1. Zusammensetzung umfassend Lactoferrin und Chondroitinsulfat und ferner umfassend bis zu 0,05 Gew.-% Vitamin D und bis zu 0,05 Gew.-% Vitamin K.

2. Zusammensetzung nach Anspruch 1, wobei Lactoferrin und Chondroitinsulfat in einem Gewichtsverhältnis von 1,5:1 bis 50:1 sind.

3. Zusammensetzung nach Anspruch 2, wobei Lactoferrin und Chondroitinsulfat in einem Gewichtsverhältnis von 1,5:1 bis 10:1, bevorzugt 1,5:1 bis 5:1 sind.

4. Zusammensetzung nach einem der Ansprüche 1-3, ferner umfassend ein Adjuvans, ausgewählt aus Kollagen, hydrolysiertem Kollagen Typ I, hydrolysiertem Kollagen Typ II, Hyaluronsäure, Dermatansulfat, Keratansulfat, Heparin, Heparansulfat und Mischungen davon.

5. Zusammensetzung nach einem der Ansprüche 1-4, wobei Vitamin D bis zu 0,01 Gew.-% bezogen auf das Zusammensetzungsgewicht ist.

6. Zusammensetzung nach einem der Ansprüche 1-5, wobei Vitamin K bis zu 0,01 Gew.-% bezogen auf das Zusammensetzungsgewicht ist.

7. Zusammensetzung nach Anspruch 6 in der Form einer Einheitsdosis, umfassend 1-1000 mg Lactoferrin, 0,1-800 mg Chondroitinsulfat, bis zu 400 mg des Adjuvans, bis zu 2200 IE Vitamin D und 1-500 µg Vitamin K.

8. Zusammensetzung nach Anspruch 7 in der Form einer Einheitsdosis, umfassend:
| | |
|---|---|
| - Lactoferrin | 50-200 mg |
| - Chondroitinsulfat | 1-100 mg |
| - Adjuvans | 50-300 mg |
| - Vitamin D3 | 1900-2100 IE |
| - Vitamin K2 | 1-200 µg. |

9. Zusammensetzung nach einem der Ansprüche 1-8 zur Verwendung bei der Vorbeugung oder Behandlung von Osteopenie sowie zur Verwendung als Coadjuvans bei der Vorbeugung von Osteoporose.

10. Nahrungsergänzungsmittel, umfassend die Zusammensetzung nach einem der Ansprüche 1-8.

## Revendications

1. Composition comprenant de la lactoferrine et du sulfate de chondroïtine, et comprenant en outre jusqu'à 0,05 % en poids de vitamine D, et jusqu'à 0,05 % en poids de vitamine K.

2. Composition selon la revendication 1, ladite lactoferrine et ledit sulfate de chondroïtine étant dans un rapport pondéral de 1,5:1 à 50:1.

3. Composition selon la revendication 2, ladite lactoferrine et ledit sulfate de chondroïtine étant dans un rapport pondéral de 1,5:1 à 10:1, de préférence de 1,5:1 à 5:1.

4. Composition selon l'une quelconque des revendications 1 à 3, comprenant en outre un adjuvant choisi parmi un collagène, un collagène hydrolysé de type I, un collagène hydrolysé de type II, l'acide hyaluronique, le sulfate de dermatane, le sulfate de kératane, l'héparine, le sulfate d'héparane et leurs mélanges.

5. Composition selon l'une quelconque des revendications 1 à 4, ladite vitamine D représentant jusqu'à 0,01 % en poids, par rapport au poids de la composition.

6. Composition selon l'une quelconque des revendications 1 à 5, ladite vitamine K représentant jusqu'à 0,01 % en poids, par rapport au poids de la composition.

7. Composition selon la revendication 6, sous la forme d'une dose unitaire comprenant 1 à 1000 mg de lactoferrine, 0,1 à 800 mg de sulfate de chondroïtine, jusqu' à 400 mg dudit adjuvant, jusqu'à 2200 IU de vitamine D et 1 à 500 µg de vitamine K.

8. Composition selon la revendication 7, sous la forme d'une dose unitaire comprenant :
| | |
|---|---|
| - lactoferrine | 50 à 200 mg |
| - sulfate de chondroïtine | 1 à 100 mg |
| - adjuvant | 50 à 300 mg |
| - Vitamine D3 | 1900 à 2100 UI |
| - Vitamine K2 | 1 à 200 µg. |

9. Composition selon l'une quelconque des revendications 1 à 8 destinée à être utilisée dans la prévention ou le traitement de l'ostéopénie, ainsi que destinée à être utilisée comme coadjuvant dans la prévention de l'ostéoporose.

10. Complément alimentaire comprenant la composition selon l'une quelconque des revendications 1 à 8.
